# EUROPEAN PATENT SPECIFICATION

(11) **EP 3 638 767 B1**
(45) Date of publication and mention of the grant of the patent: **03.03.2021**
(21) Application number: 18745655.3
(22) Date of filing: 13.06.2018
(51) Int. Cl.: C12M 1/107, C12M 1/00

(54) **A BIOGAS REACTOR**
BIOGASREAKTOR
RÉACTEUR DE BIOGAZ

(30) Priority: 13.06.2017 FI 20175547
(43) Date of publication of application: 22.04.2020
(73) Proprietor: Demeca OY, 86600 Haapavesi (FI)
(72) Inventor: HAAPAKOSKI, Tomi, 86600 Haapavesi (FI); VINKKI, Pekka, 86600 Haapavesi (FI); LOHI, Juho, 70780 Kuopio (FI); VINKKI, Tuomas, 86600 Haapavesi (FI)
(74) Representative: Berggren Oy, Helsinki & Oulu
(86) International application number: PCT/FI2018/050458
(87) International publication number: WO 2018/229340

(56) References cited:
- EP-A1- 1 867 712
- CN-A- 101 845 388
- CN-U- 201 485 455
- CN-U- 201 825 951
- DE-U1-202008 003 542
- DE-U1-202012 104 130

## Description

The invention relates to a biogas reactor, which has a floor and walls, which floor and walls form a substantially liquid-proof basin, a gas-permeable intermediate floor which substantially completely covers the basin and a roof structure above the intermediate floor.

Biogas can be produced by digesting organic biomass, such as separated sludge manure, in anoxic conditions. In biogas production facilities, the digesting is done in an airtight biogas reactor manufactured for this purpose. A conventional biogas reactor has a water-proof concrete floor slab, on top of which concrete walls are built, so that the concrete slab and walls together form a liquid-proof basin. A gas-proof gas hood is attached to the upper edge of the walls, which forms the roof of the bioreactor. The biomass is led into the basin, in the digesting process occurring in which biogas containing e.g. methane is formed. Typically, the biomass to be digested in the biogas reactor is sludge in liquid form, which can be moved by pumping. The biogas formed fills the space between the surface of the biomass in the basin and the gas hood, whereby the gas hood rises into a dome-like shape due to the gas pressure. As the biogas pressure sinks, for example in connection with gas removal, the hood sinks down. In order for the gas hood not to sink down onto the biomass sludge in the basin, a net covering the entire basin has been set up at the level of the upper edge of the walls. In the middle of the basin there is a support pillar for carrying the net, from which pillar straps emerge radially, which straps are attached by their other end to the upper edge of the walls. One such biogas reactor is presented in publication EP 1867712 A1.

Several problems are associated with known biogas reactors. In addition to combustible gases, such as methane, also among others poisonous hydrogen sulphide is formed in the biogas production process. Hydrogen sulphide corrodes the structures of the biogas reactor, wherefore the steel support pillars and reinforcements of the concrete structures must be made of a more expensive acid-proof or stainless steel. Due to the hydrogen sulphide contained in the biogas, gas motors which can withstand corrosive fuel, which are substantially more expensive than conventional motors, must be used as motors rotating the electricity generators in biogas facilities. The gas hood of the biogas reactor, made from a thin film, isolates heat poorly, which increases the heating needs of the biomass.

An object of the invention is to provide a biogas reactor, by which the problems relating to known biogas reactors can be reduced. The objects of the invention are obtained with a biogas reactor, which is characterized by what is presented in the independent claims. Some advantageous embodiments of the invention are presented in the dependent claims.

The invention relates to a biogas reactor, which has a floor and a wall, which form a substantially liquid-proof basin. The basin functions as a digesting space for the biomass to be led into the biogas reactor. The biogas reactor further has a gas-permeable intermediate floor substantially completely covering the basin and a roof structure above the intermediate floor. Said intermediate floor comprises support beams, which to their material are made of wood. The intermediate floor is situated in the space between the biomass and the roof structure, in which space corrosive hydrogen sulphide is formed. The support beams made of solid wood do not have glue seams and/or joints, which could break in challenging conditions.

In one advantageous embodiment of the bioreactor according to the invention said intermediate floor further comprises a porous thermal insulation layer. The thermal insulation layer slows down the escape of heat from the bioreactor, whereby the temperature of the biomass remains at an optimal level for biogas production with very little external heating.

In a second advantageous embodiment of the bioreactor according to the invention, said thermal insulation layer is at least partly made of wood fibre material. The thermal insulation layer is advantageously at least partly made of cutter shavings. Alternatively or additionally, the thermal insulation layer can be at least partly made of granular polyurethane. Is has been found that a porous thermal insulation layer, particularly a cutter shaving layer, decreases the amount of hydrogen sulphide formed in the biogas reactor. It is assumed that this is partly due to the fact that the porous thermal insulation layer offers a breeding ground for bacteria which use hydrogen sulphide as nutrition. Small amounts of air can be fed with air pipes into the space between the thermal insulation layer and biomass, whereby the oxygen in the air and the hydrogen sulphide formed in the biogas process react together and the sulphur in the hydrogen sulphide is precipitated into sulphur on the porous surfaces of the thermal insulation layer. The precipitated sulphur can fall into the biomass and exit the reactor in connection with the removal of the used biomass. A thermal insulation layer made from cutter shavings offers a very large surface for the occurrence of the above-mentioned reactions, whereby a small amount of air fed into the thermal insulation layer does not disturb the production of biogas requiring anoxic conditions.

In a third advantageous embodiment of the bioreactor according to the invention there is a support pillar in the basin, which pillar has a first end, which is supported on the floor. The support beams have a first end, by which the support beam is supported on the wall, and a second end, by which the support beam is supported on the support pillar.

Still another advantageous embodiment of the bioreactor according to the invention comprises a gas-proof gas hood, which is supported on the second end of the support pillar.

In still another advantageous embodiment of the bioreactor according to the invention, said wall has an inner surface, an upper edge and a lower edge, and the inner surface of the walls have a gas-proof protective film extending from the upper edge to the lower edge.

In still another advantageous embodiment of the bioreactor according to the invention, the gas hood has an edge and the protective film has an upper edge and the upper edge of the protective film and the edge of the gas hood are connected together in a gas-proof manner. The protective film advantageously has a lower edge, which is connected to the floor in a liquid-proof manner.

An advantage of the invention is that the durability of the structures of the inner parts of the biogas reactor is improved and the service life is increased.

An advantage of one embodiment of the invention is that it improves the operating conditions of microbes producing biogas and thus increases the amount of biogas formed.

An advantage of one embodiment of the invention is further that the amount of hydrogen sulphide in the biogas is reduced, which makes easier the use of the biogas as fuel in gas motors.

In the following, the invention will be described in detail. In the description, reference is made to the enclosed drawings, in which
figure 1 shows as an example a biogas reactor according to the invention as partly cut open and
figure 2 shows a wall of the biogas reactor shown in figure 1 as a cross-sectional view.

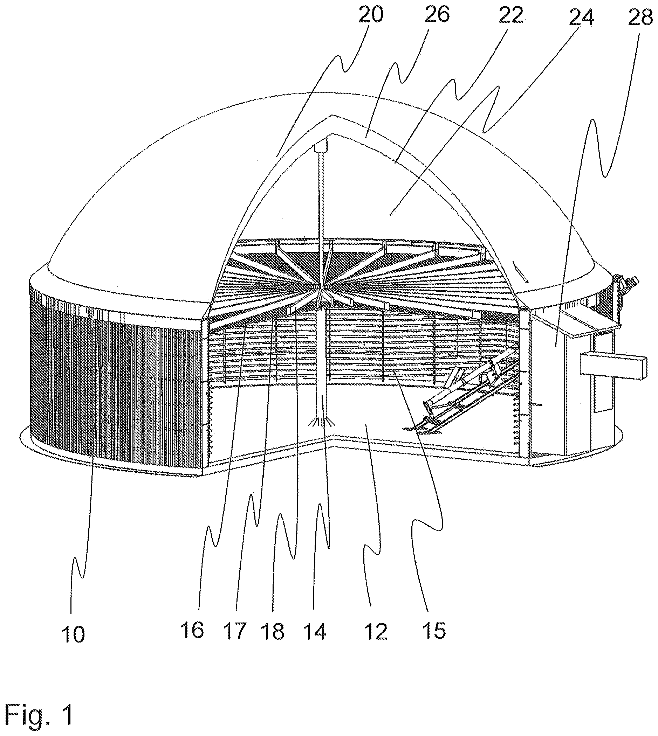

Figure 1 shows as an example a biogas reactor according to the invention seen from the outside. The biogas reactor has a roof structure, a wall 10 and a floor 12. In figure 1 a part of the roof structure, wall and floor have been removed in order to better bring out the structure of the inner part of the biogas reactor. The floor of the biogas reactor is to its shape a round fibre concrete slab. The walls of the biogas reactor are built from wall elements, which are erected on a thermal insulation layer 11 formed on top of a levelled soil layer, so that they form a solid, substantially cylindrical outer wall. The walls and floor form a basin, into which the biomass in liquid or paste-like form, typically sludge manure, to be used as raw material in biogas production, is led. On the inner surface of the walls there is a heating pipe system 15, with the aid of which the biomass in the biogas reactor is heated to a temperature optimal for gas production.

In the centre of the space delimited by the walls there is a support pillar 14, which is supported by its first end on the floor. The material of the support pillar is hot-dip galvanized steel. At the level of the upper edge of the walls there is an intermediate floor 16, the bearing structure of which is formed from solid wood support beams 18. The support beams are supported by their first end on the upper edge of the wall and by their second end on the support pillar. Wooden sparse boarding 17 is attached to the lower edge of the support beams. The width of the gap between the boards of the sparse boarding can be 5-20 mm. On top of the sparse boarding there is an approximately 30 cm thick layer of cutter shavings, which forms a gas-permeable thermal insulation layer in the intermediate floor (the thermal insulation layer is not shown in the figures). On the outer surface of the wall there is a control room 28, which contains the machines and devices that control and monitor the operation of the biogas reactor.

The roof structure of the biogas reactor comprises a cover tarpaulin 20 protecting from the weather and a gas-proof gas hood 22 underneath the cover tarpaulin. The gas hood and cover tarpaulin are supported by their edges in a gas-proof manner to the upper edge of the wall 10. The gas hood is a film, which rises into a dome-like shape due to gas pressure formed beneath it. The second end of the support pillar supports the gas hood by its middle part, so that when the pressure sinks, the gas hood cannot fall down onto the intermediate floor. The space between the gas hood and the intermediate floor is called the gas space 24. The biogas formed in the biogas reactor is collected in the gas space, from where it is led along a collecting pipe for further processing. Between the cover tarpaulin and gas hood there is an air space 26, which improves the thermal insulation ability of the roof structure. The air pressure in the air space is upheld with a compressor in the control room 28, so that the cover tarpaulin continuously retains its dome-like shape, regardless of the magnitude of the pressure prevailing in the gas space.

The biogas reactor further comprises feeding means for feeding in biomass, discharge means for removing used biomass, i.e. reject, and a mixing means for mixing the biomass in the reactor. However, these are not within the scope of this invention, thus they are not described further in this connection.

The size of the biogas reactor can be selected according to the amount and quality of the biomass to be processed and the biogas production targets. The diameter of the biogas reactor can be 12-16 meters and the height of the wall 3-5 meters. The height of the bioreactor, i.e. the distance from the ground level to the highest point of the cover tarpaulin can be 8-15 meters. Preferably, the diameter of the biogas reactor is about 15.6 meters, the height of the wall about 4.4. meters and the total height about 9.2 meters.

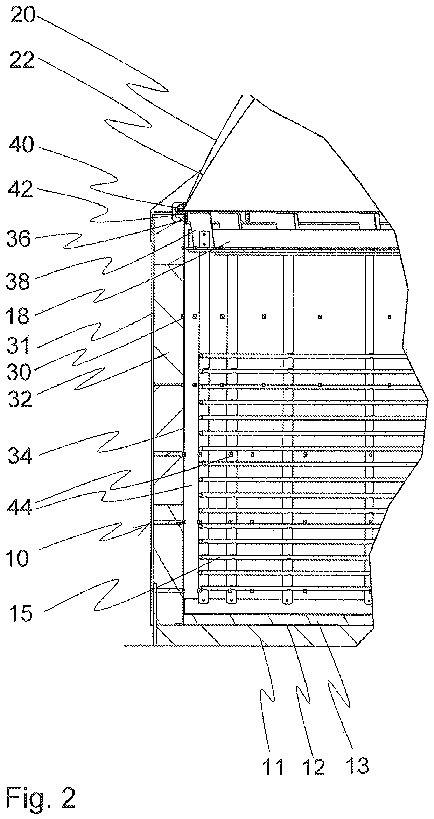

Figure 2 shows a wall of the biogas reactor shown in figure 1 as a cross-sectional view. The floor 12 of the biogas reactor comprises a bearing bottom slab 13, which is made of fibre concrete and cast on a durable insulation layer 11. The wall 10 of the bioreactor is built on the insulation layer, so that the lower edge of the wall settles against the upper surface of the insulation layer. The wall comprises a bearing steel inner mantle 30 with an element structure, a thermal insulation 32 and an outer shell 31. The thermal insulation is attached with a comb structure to the steel elements of the inner mantle. The outer shell is made of corrugated metal, which is attached to the comb structure binding the insulation. On the inner surface of the upper edge of the wall there is a corner support 36 the length of the entire upper edge of the wall, to which beam supports 38 are attached for supporting the ends of the support beams 18. A rain water hem 46 circles the upper edge of the wall, which is supported by one edge in a water-proof manner to the surface of the cover tarpaulin 20.

On the inner surface of the wall there is a protective film 34, which extends from the upper edge of the wall to the lower edge of the wall over the entire length of the wall. The upper edge of the protective film is folded on top of the upper edge of the wall superimposed by the edge of the cover tarpaulin 20 and the edge of the gas hood 22. The overlapping edges are pressed tightly against the upper edge of the wall with the aid of a mounting rim 40 circling the upper edge of the wall. The mounting rim is attached in place in the upper edge of the wall with C-shaped attaching means 42. The mounting rim, attaching means and the overlapping edges of the cover tarpaulin 20, gas hood 22 and protective film 34 remain hidden underneath the rain water hem 46. The lower edge of the protective film extends in the lower edge of the wall to the upper surface of the insulation layer and its edges are folded onto the upper surface of the insulation layer. The lower edge of the protective film thus gets pinned between the bottom slab 13 to be cast on the insulation layer 11 after the installation of the walls and the upper surface of the insulation layer. The joint between the lower edge of the protective film and the floor thus becomes gas-proof. Inside the protective film, there are at a distance from each other vertical batten-like brackets 44, to which the biomass heating pipes 15 are attached.

## Claims

1. A biogas reactor, which has a floor (12) and a wall (10), which floor (12) and wall (10) form a substantially liquid-proof basin, a gas-permeable intermediate floor (16) substantially completely covering the basin and a roof structure above the intermediate floor (16), **characterized in that** said intermediate floor (16) comprises a porous thermal insulation layer and support wooden beams (18), and in the basin there is a support pillar (14), which has a first end, which is supported on the floor (12), and said support beams (18) have a first end, by which the support beam (18) is supported on the wall (10), and a second end, by which the support beam (18) is supported on the support pillar (14).

2. The biogas reactor according to claim 1, **characterized in that** said thermal insulation layer is at least partly made of wood fibre material.

3. The biogas reactor according to claim 1 or 2, **characterized in that** the thermal insulation layer is at least partly made of cutter shavings.

4. The biogas reactor according to any of the claims 1-3, **characterised in that** the thermal insulation layer is at least partly made of granular polyurethane.

5. The biogas reactor according to any of the claims 1-4, **characterized in that** said roof structure comprises a gas-proof gas hood (22), which gas hood (22) is supported on the second end of the support pillar (14).

6. The biogas reactor according to any of the claims 1-5, **characterized in that** said wall (10) has an inner surface, an upper edge and a lower edge, and the inner surface of the walls have a gas-proof protective film (34) extending from the upper edge to the lower edge.

7. The biogas reactor according to claim 6, **characterized in that** the gas hood (22) has an edge and the protective film (34) has an upper edge and the upper edge of the protective film (34) and the edge of the gas hood (22) are connected together in a gas-proof manner.

8. The biogas reactor according to claim 6 or 7, **characterized in that** the protective film (34) has a lower edge, which is connected to the floor (12) in a liquid-proof manner.

## Patentansprüche

1. Biogasreaktor, der einen Boden (12) und eine Wand (10) aufweist, wobei Boden (12) und Wand (10) ein im Wesentlichen flüssigkeitsdichtes Becken bilden, wobei ein gasdurchlässiger Zwischenboden (16) und eine Dachkonstruktion über dem Zwischengeschoss (16) das Becken im Wesentlichen vollständig abdecken, **dadurch gekennzeichnet, dass** der Zwischenboden (16) eine poröse Wärmedämmschicht und Stützholzbalken (18) umfasst,
und es in dem Becken eine Stützsäule (14) gibt, die ein erstes Ende aufweist, das auf dem Boden (12) abgestützt ist, und die Stützbalken (18) ein erstes Ende, durch das der Stützbalken (18) auf der Wand (10) abgestützt ist, und ein zweites Ende, durch das der Stützbalken (18) auf der Stützsäule (14) abgestützt ist, aufweisen.

2. Biogasreaktor nach Anspruch 1, **dadurch gekennzeichnet, dass** die Wärmedämmschicht zumindest teilweise aus Holzfasermaterial besteht.

3. Biogasreaktor nach Anspruch 1 oder 2, **dadurch gekennzeichnet, dass** die Wärmedämmschicht zumindest teilweise aus Fräserspänen besteht.

4. Biogasreaktor nach einem der Ansprüche 1 bis 3, **dadurch gekennzeichnet, dass** die Wärmedämmschicht zumindest teilweise aus körnigem Polyurethan besteht.

5. Biogasreaktor nach einem der Ansprüche 1 bis 4, **dadurch gekennzeichnet, dass** die Dachkonstruktion eine gasdichte Gashaube (22) umfasst, wobei die Gashaube (22) am zweiten Ende der Stützsäule (14) abgestützt ist.

6. Biogasreaktor nach einem der Ansprüche 1 bis 5, **dadurch gekennzeichnet, dass** die Wand (10) eine Innenfläche, eine Oberkante und eine Unterkante aufweist und die Innenfläche der Wände eine gasdichte Schutzfolie (34) aufweist, die sich von der Oberkante zur Unterkante erstreckt.

7. Biogasreaktor nach Anspruch 6, **dadurch gekennzeichnet, dass** die Gashaube (22) eine Kante aufweist und die Schutzfolie (34) eine Oberkante und die Oberkante der Schutzfolie (34) aufweist und der Rand der Gashaube (22) gasdicht miteinander verbunden sind.

8. Biogasreaktor nach Anspruch 6 oder 7, **dadurch gekennzeichnet, dass** die Schutzfolie (34) eine Unterkante aufweist, die flüssigkeitsdicht mit dem Boden (12) verbunden ist.

## Revendications

1. Réacteur de biogaz, qui a un plancher (12) et une paroi (10), lesquels plancher (12) et paroi (10) forment une cuvette sensiblement étanche aux liquides, un plancher intermédiaire perméable aux gaz (16) recouvrant sensiblement complètement la cuvette et une structure de toit au-dessus du plancher intermédiaire (16), **caractérisé en ce que** ledit plancher intermédiaire (16) comprend une couche d'isolation thermique poreuse et des poutres en bois de support (18),
et dans la cuvette il y a un pilier de support (14), qui a une première extrémité, qui est supportée sur le plancher (12), et lesdites poutres de support (18) ont une première extrémité, par laquelle la poutre de support (18) est supportée sur la paroi (10), et une seconde extrémité, par laquelle la poutre de support (18) est supportée sur le pilier de support (14).

2. Réacteur de biogaz selon la revendication 1, **caractérisé en ce que** ladite couche d'isolation thermique est au moins en partie constituée d'un matériau en fibres de bois.

3. Réacteur de biogaz selon la revendication 1 ou 2, **caractérisé en ce que** la couche d'isolation thermique est au moins en partie constituée de copeaux de coupe.

4. Réacteur de biogaz selon l'une quelconque des revendications 1 à 3, **caractérisé en ce que** la couche d'isolation thermique est au moins en partie constituée de polyuréthane granulaire.

5. Réacteur de biogaz selon l'une quelconque des revendications 1 à 4, **caractérisé en ce que** ladite structure de toit comprend une capote de dégagement des gaz (22) étanche aux gaz, laquelle capote de dégagement des gaz (22) est supportée sur la seconde extrémité du pilier de support (14).

6. Réacteur de biogaz selon l'une quelconque des revendications 1 à 5, **caractérisé en ce que** ladite paroi (10) a une surface interne, un bord supérieur et un bord inférieur, et la surface interne des parois a un film protecteur (34) étanche aux gaz s'étendant du bord supérieur au bord inférieur.

7. Réacteur de biogaz selon la revendication 6, **caractérisé en ce que** la capote de dégagement des gaz (22) a un bord et le film protecteur (34) a un bord supérieur et le bord supérieur du film protecteur (34) et le bord de la capote de dégagement des gaz (22) sont reliés ensemble de manière étanche aux gaz.

8. Réacteur de biogaz selon la revendication 6 ou 7, **caractérisé en ce que** le film protecteur (34) a un bord inférieur, qui est relié au plancher (12) de manière étanche aux liquides.
